# EUROPÄISCHE PATENTSCHRIFT

(11) **EP 0 931 046 B1**
(45) Veröffentlichungstag und Bekanntmachung des Hinweises auf die Patenterteilung: **27.06.2001**
(21) Anmeldenummer: 97919009.7
(22) Anmeldetag: 29.08.1997
(51) Int. Cl.: C07C 41/00, C07C 43/00

(54) **VERFAHREN ZUR HERSTELLUNG VON n-BUTYLALKYLETHERN**
PROCESS FOR PRODUCING n-BUTYLALKYL ETHERS
PROCEDE POUR LA PREPARATION DE n-BUTYLALKYLETHERS

(30) Priorität: 17.09.1996 DE 19637895
(43) Veröffentlichungstag der Anmeldung: 28.07.1999
(73) Patentinhaber: BASF AKTIENGESELLSCHAFT, 67056 Ludwigshafen (DE)
(72) Erfinder: KANAND, Jürgen, D-67098 Bad Dürkheim (DE); RÖPER, Michael, D-67157 Wachenheim (DE)
(86) Internationale Anmeldenummer: EP9704703
(87) Internationale Veröffentlichungsnummer: WO9812164

(56) Entgegenhaltungen:
- DE-A- 4 400 837
- US-A- 3 670 029
- US-A- 3 769 352
- US-A- 4 843 180

## Beschreibung

Die Erfindung betrifft ein Verfahren zur Herstellung n-Butylalkylethern durch Umsetzung von Butadien mit Alkoholen zu einem Gemisch aus 1- und 3-Alkoxybuten, Isomerisierung des 3-Alkoxybutens zu 1-Alkoxybuten und Hydrierung des 1-Alkoxybutens.

n-Butylalkylether sind Produkte der chemischen Industrie und finden vielseitige Verwendung. So wird beispielsweise Di-n-butylether als Lösungsmittel bei verschiedenen natürlichen und synthetischen Harzen, Fetten, Ölen und Grignard-Reaktionen eingesetzt. In Mischung mit Ethanol oder Butanol findet D-n-butylether ferner Anwendung zur Lösung von Ethylcellulose.

Di-n-butylether wird großtechnisch praktisch ausschließlich durch die Dehydratisierung von n-Butanol nach verschiedenen Verfahren hergestellt, bei denen die Umsetzung z.B. in Gegenwart von Schwefelsäure oder Katalysatoren wie Eisenchlorid, Kupfersulfat, Kieselgel oder Aluminiumoxid durchgeführt wird (Kirk-Othmer: Encyclopedia of Chemical Technology, 4. Aufl., Band 9, S. 860 - 876, John Wiley & Sons, New York 1992; Ullmann's Encyclopedia of Industrial Chemistry, 5. Aufl., Band A 10, S. 23 - 34, Verlag Chemie Weinheim). Diese Verfahren, insbesondere die Dehydratisierung mit Hilfe der heterogenen Katalysatoren erfordern hohe Reaktionstemperaturen, die zu Nebenreaktionen führen. Desweiteren können bei den homogenen Verahren mit Schwefelsäure oder Metallsalzen nur Alkohole mit geringer Kettenlänge (C₁ - C₃) erfolgreich dehydratisiert werden.

Unsymmetrische n-Butylalkylether, beispielsweise 2 - (n-Butoxy)ethanol oder 1-(n-Butoxy)propanol-2, die als Lösungsmittel in der Lackindustrie dienen, werden üblicherweise durch Umsetzung von n-Butanol mit Ethylenoxid bzw. Propylenoxid (Encyclopedia of Chemical Processing and Design, Band 20, S. 258 - 274, Marcel Dekker , Inc, New York and Basel 1984) hergestellt. Aus US 4 843 180 ist z.B. bekannt, daß Diene mit Hydroxyverbindungen zu ungesättigten Butylethern reagieren, die in einer weiteren Stufe zu Gemischen aus linearen und überwiegend verzweigten gesättigten Ethern hydriert werden können.

Ziel der Erfindung war es nun, ein Verfahren zur Herstellung von n-Butylalkylethern ausgehend von dem leicht verfügbaren Butadien vorzuschlagen. Obwohl 1,3-Butadien in großen Mengen zur Verfügung steht und ein sehr preiswerter Rohstoff ist, wurde bislang noch kein großtechnisch anwendbares Verfahren zur Herstellung von n-Butylalkylethern auf der Basis von 1,3-Butadien oder Butadien-haltigen Kohlenwasserstoffgemischen bekannt. Ein Grund hierfür ist sowohl in der Neigung des 1,3-Butadiens zu Dimerisierungs- und Polymerisationsreaktionen als auch die Bildung von Gemischen aus 1,2- und 1,4-Addukten bei Additionsreaktionen zu sehen. Ursächlich für dieses chemische Verhalten ist das Vorliegen zweier konjugierter Doppelbindungen im 1,3-Butadienmolekül (Kirk-Othmer: Encyclopedia of Chemical Technology, 4. Aufl., Band 4, S. 676 - 683, John Wiley & Sons, New York 1992).

So ist aus zahlreichen Literaturstellen die Umsetzung von Butadien mit Alkoholen und auch die Hydrierung der Butenylether beschrieben, ohne daß bisher ein technisch durchführbares Verfahren gefunden wurde.

Aus WO 95/19334 ist insbesondere bereits bekannt und im einzelnen beschrieben, Alkohole an Butadien zu den Alkylbutenylethern anzulagern und die 3-Alkoxybuten-(1)-Verbindung zu isomerisieren. Dort findet jedoch eine weitere Isomerisierung zum Enolether statt. Eine Hydrierung der 1-Alkoxybutene- (2) ist nicht in Betracht bezogen und beschrieben.

Für die Hydrierung von allgemein Allylethern zu n-Butylalkylethern sind nur wenige Beispiele bekannt. In US 3 769 352 und US 3 670 029 und US 4 843 180 wird lediglich erwähnt, daß die ungesättigten Ether hydriert werden können.

Der vorliegenden Erfindung lag somit die Aufgabe zugrunde, ein großtechnisch anwendbares, wirtschaftliches Verfahren zur Herstellung von n-Butylalkylethern zu finden, das es ermöglicht, diese Produkte mit hoher Ausbeute und Selektivität herzustellen.

Diese Aufgabe wurde gelöst mit einem Verfahren zur Herstellung von n-Butylalkylethern, wobei man
a) 1,3-Butadien oder Butadien-haltige Kohlenwasserstoffgemische mit einem Alkohol der Formel I

   ROH I,

   in der der Rest R eine unsubstituierte oder mit 1 bis 2 C₁-bis C₁₀-Alkoxy- oder Hydroxygruppen substituierte C₂- bis C₂₀-Alkyl-, Alkenyl-, Cycloalkyl- oder Cycloalkenyl-, eine C₆-bis C₁₀-Aryl- oder eine C₇- bis C₁₁-Aralkylgruppe oder die Methylgruppe ist, bei erhöhter Temperatur und bei erhöhtem Druck in Gegenwart einer Brönsted-Säure oder in Gegenwart eines Komplexes eines Elementes aus der Gruppe Ib, VIIb oder VIIIb des Periodensystems der Elemente mit Phosphor- oder Stickstoff-haltigen Liganden zu einem Gemisch der Addukte der Formeln II 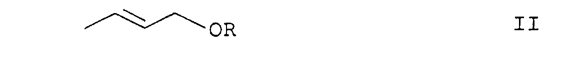 und III 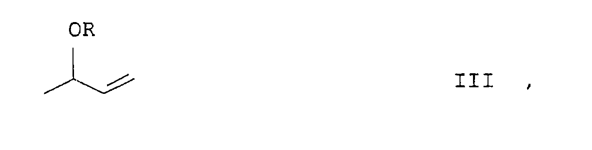 in der R die obengenannten Bedeutungen hat, umsetzt,
b) die Addukte der Formel II und III trennt,
c) das Addukt III zum Addukt II in Gegenwart eines Katalysators wie für (a) angegeben, isomerisiert, und
d) das Addukt II in Gegenwart eines homogenen oder heterogenen Übergangsmetallelement-Katalysators in flüssiger Phase oder in Gegenwart eines heterogenen, Übergangsmetallelement-haltigen Katalysators in der Gasphase zum n-Butylalkylether der Formel IV 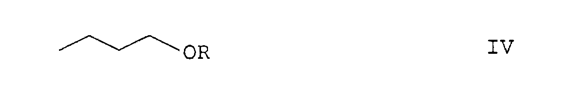 hydriert.

Das erfindungsgemäße Verfahren besteht somit aus 4 Stufen, wobei die Teilreaktionen (a) und (c) wahlweise einzeln und nacheinander oder nebeneinander oder in einer Verfahrensstufe durchgeführt werden. In letzterem Fall verläuft die Isomerisierung des Addukts III zum Addukt II gemäß Teilreaktion c) nach Rückführung des Addukts III in die Verfahrensstufe der Addition des Alkohols ROH an 1,3-Butadien simultan mit der Addition gemäß Teilreaktion (a) .

In der Regel ist der in einer Anlageneinheit für die Teilreaktion (a) verwendete Katalysator in der Lage, unter den dort angewandten Reaktionsbedingungen die Isomerisierung des Addukts III zum Addukt II und die Addition des Alkohols I an 1,3-Butadien zu katalysieren, so daß keine strenge räumliche Trennung des Ablaufs dieser Teilreaktionen vorliegt. Allerdings kann eine Anlageneinheit für die Stufe (a) sowohl einen einzigen Reaktor als auch mehrere, in Reihe geschaltete Reaktoren umfassen, die mit dem gleichen oder gegebenenfalls mit verschiedenen Katalysatoren befüllt sind und in der gleichen Betriebsweise und bei gleichen oder verschiedenen Temperatur- und Druckbedingungen betrieben werden. Als Betriebsweise wird dabei jeweils das Arbeiten in flüssiger Phase unter Anwendung eines Homogenkatalysators oder Heterogenkatalysators oder in Ausnahmefällen, das Arbeiten in der Gasphase verstanden.

Im folgenden wird das erfindungsgemäße Verfahren näher erläutert:

In Stufe (a) wird 1,3-Butadien oder ein Butadien-haltiges Kohlenwasserstoffgemisch in Gegenwart eines Katalysators mit dem Alkohol ROH I gemäß Gleichung (1) 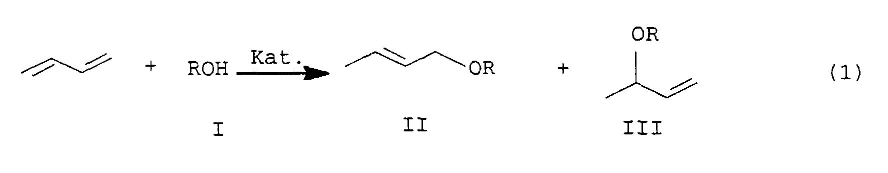 zum 1,4-Addukt der Formel II und dem 1,2-Addukt der Formel III umgesetzt. Im entstandenen 1,4-Addukt II kann die Doppelbindung sowohl in der cis- als auch in trans-Form vorliegen; dies ist für den weiteren Ablauf des Verfahrens aber nicht erheblich. Die Addukte II und III entstehen dabei je nach Reaktionsbedingungen und angewandtem Katalysator im allgemeinen in einem Molverhältnis von 1:1 bis 1:3.

Die Art des in der Umsetzung eingesetzten Alkohols ROH I ist in der Regel für das Verfahren nicht kritisch. Es können sowohl primäre als auch sekundäre Alkohole verwendet werden, bevorzugt werden aber primäre Alkohole eingesetzt. Es können sowohl aliphatische, cycloaliphatische, aromatische als auch araliphatische Alkohole benutzt werden, vorzugsweise finden aliphatische und araliphatische Alkohole Anwendung. Im allgemeinen werden Alkohole ROH I im erfindungsgemäßen Verfahren verwendet, in denen der Rest R eine C₁- bis C₂₀-Alkyl-, C₂- bis C₁₀-Alkenyl-, z.B. die But-2-enyl-, vorzugsweise eine C₁- bis C₄-Alkyl-, insbesondere die n-Butylgruppe, eine C₆- bis C₁₀-Aryl-, vorzugsweise die Phenylgruppe oder eine C₇- bis C₁₁-Aralkyl-, vorzugsweise die Benzylgruppe ist. Die Reste R können gegebenenfalls mit Substituenten wie C₁- bis C₁₀-Alkoxy- und/oder Hydroxyl-Gruppen substituiert sein. Als Alkohole ROH I können somit auch Diole, vorzugsweise Ethylenglykol oder 1,2-Propandiol, oder Triole oder Alkoxyalkohole verwendet werden. Selbstverständlich können auch Alkohole mit einer höheren Anzahl an Kohlenstoffatomen eingesetzt werden. Da solche höhere Alkohole in der Regel teurer sind als niedere Alkohole, werden aus wirtschaftlichen Gründen niedere Alkohole bevorzugt verwendet. Von den genannten Alkoholen ist n-Butanol zur Herstellung von Di-n-butylether bevorzugt.

Als Katalysatoren können in Stufe (a) eine Vielzahl von Katalysatoren eingesetzt werden, beispielsweise Brönsted-Säuren oder Komplexe von Übergangsmetallen aus den Gruppen Ib, VIIb oder VIIIb des Periodensystems der Elemente, insbesondere von Palladium und Nickel.

Als Brönsted-Säuren kommen beispielsweise herkömmliche, nicht oxidierende Brönsted-Säuren, wie Halogenwasserstoffsäuren, z.B. Salzsäure, Schwefelsäure, Phosphorsäure, Perchlorsäure, Flußsäure, Tetrafluoroborsäure, Methansulfonsäure oder Toluolsulfonsäure, vorzugsweise aber feste Brönsted-Säuren, insbesondere organische oder anorganische Kationentauscher, in Betracht.

Die Komplexe, insbesondere Phosphinkomplexe der Übergangsmetallelementkatalysatoren, die ein Element aus der Gruppe Ib, VIIb oder VIIIb des Periodensystems der Elemente, wie Kupfer, Nickel, Rhodium, Palladium, Platin, Rhenium oder Iridium, vorzugsweise Palladium oder Nickel, enthalten, können homogen im Reaktionsmedium gelöst oder heterogen vorliegen.

Die Herstellung der Alkylbutenylether erfolgt generell nach den Angaben der WO 95/19334 sowohl in Bezug auf die verwendeten Katalysatoren, als auch die Verfahrensbedingungen, so daß auf diese Literatur und die darin zitierte Vorliteratur verwiesen und ausdrücklich auf die Angaben dieser Anmeldeschrift Bezug genommen wird und die dort gemachten Angaben als hier inkorporiert gelten sollen.

Das Molverhältnis Alkohol/1,3-Butadien kann aus einem weiten Bereich gewählt werden. Im allgemeinen wird ein Molverhältnis Alkohol ROH/1,3-Butadien von 0,5:1 bis 10:1 vorzugsweise von 1:1 bis 3,0:1 und besonders bevorzugt von 1,5:1 bis 2,5:1 angewandt. Zweckmäßigerweise wird der Alkohol ROH immer in einem mindestens einmolaren Überschuß angewandt, da dadurch eine höhere Ausbeute erzielt werden kann. Die Umsetzung des Alkohols ROH I mit 1,3-Butadien wird bei Durchführung des Verfahrens in flüssiger Phase im allgemeinen bei Temperaturen von 20 bis 150°C, vorzugsweise von 50 bis 120°C, insbesondere von 60 bis 110°C und bei einem Druck von im allgemeinen 1 bis 100 bar, vorzugsweise von 5 bis 50 bar, insbesondere von 10 bis 30 bar, vorgenommen. Zweckmäßigerweise wird der Druck so gewählt, daß das 1,3-Butadien oder die Butadien-haltigen Kohlenwasserstoffgemische bei der angewandten Reaktionstemperatur flüssig sind. Die Anwendung eines höheren Drucks ist möglich. Die angewandte Reaktionstemperatur wird zweckmäßigerweise hinsichtlich des jeweils verwendeten Katalysators in einem Vorversuch optimiert.

Im allgemeinen wird das Alkohol ROH/1,3-Butadien-Gemisch mit einer Raumgeschwindigkeit von 0,01 bis 0,5 g/cm³·h, vorzugsweise von 0,05 bis 0,4 g/cm³·h und besonders bevorzugt von 0,10 bis 0,25 g/cm³·h durch das Katalysatorfestbett geleitet. Der Zusatz eines Lösungsmittels zum Reaktionsgemisch ist möglich, im allgemeinen aber nicht erforderlich, da der eingesetzte Alkohol als auch die Addukte II und III auch als Lösungsmittel fungieren können. Die Verweilzeit des Alkohol ROH/1,3-Butadiengemisches im Reaktor beträgt im allgemeinen 1 bis 6 Stunden und ist in der Regel von der angewandten Reaktionstemperatur abhängig.

Wird die Addition des Alkohols ROH I an 1,3-Butadien oder die Butadien-haltigen Kohlenwasserstoffgemische in der Gasphase durchgeführt, werden im allgemeinen Temperaturen von weniger als 120°C und ein Druck von im allgemeinen von weniger als 20 bar angewandt. Gewünschtenfalls kann das Reaktionsgas mit einem unter den Reaktionsbedingungen inerten Gas, z.B. Stickstoff, vermischt werden, im allgemeinen wird das Reaktionsgas unverdünnt eingesetzt.

Anstelle von reinem 1,3-Butadien können auch 1,3-Butadien-haltige Kohlenwasserstoffgemische als Rohstoff eingesetzt werden. Derartige Kohlenwasserstoffgemische fallen beispielsweise als sogenannter C₄-Schnitt in Steamcrackern an. Zweckmäßigerweise werden diese Kohlenwasserstoffgemische vor ihrem Einsatz von gegebenenfalls darin enthaltenen acetylenischen oder allenischen Kohlenwasserstoffen durch deren partielle Hydrierung (Weissermel, Arpe: Industrielle Organische Chemie; 3. Auflage, VCH Verlagsgesellschaft, Weinheim 1988) und, falls erwünscht, von Isobuten befreit. Die 1,3-Butadien-haltigen Kohlenwasserstoffgemische können sodann in analoger Weise wie reines 1,3-Butadien in die Teilreaktion (a) eingetragen werden. Zweckmäßigerweise werden die in diesen Kohlenwasserstoffgemischen enthaltenen gesättigten oder monoolefinischen Kohlenwasserstoffe, die bei der Umsetzung in der Teilreaktion (a) nicht reagiert haben, aus dem Reaktionsaustrag entfernt, beispielsweise mittels eines Gas-Flüssigkeit-Abscheiders oder einer Druckdestillation. Die bei der Umsetzung dieser Kohlenwasserstoffgemische in der Teilreaktion (a) erhaltenen Addukte der Formeln II und III können auf die gleiche Weise zu den n-Butylalkylethern weiterverarbeitet werden, wie die mit reinem 1,3-Butadien in Teilreaktion (a) erzeugten Addukte II und III.

Der Reaktionsaustrag aus der Teilreaktion (a) des erfindungsgemäßen Verfahrens enthält im allgemeinen neben nicht umgesetztem 1,3-Butadien oder olefinischen Kohlenwasserstoffen die Addukte der Formeln II und III sowie gegebenenfalls, insbesondere bei Anwendung von Brönsted-Säuren als Katalysatoren, mehrere Isomere des betreffenden Alkoxyoctadiens, von denen im folgenden unter der Sammelbezeichnung Alkoxyoctadien gesprochen wird. Das Alkoxyoctadien entsteht bei der Addition des Alkohols ROH I an 1,3-Butadien in einer Nebenreaktion, in der zunächst 1,3-Butadien zu Octatrien dimerisiert, an das sich nachfolgend der Alkohol ROH I unter Bildung des Alkoxyoctadiens addiert. Neben diesen Bestandteilen kann der Reaktionsaustrag aus Teilreaktion (a) noch geringe Mengen an anderen Nebenprodukten enthalten, beispielsweise Octatrien, Vinylcyclohexen, Alkoxydodecatriene, entstanden durch Trimerisierung des 1,3-Butadiens zu Dodecatetraen und nachfolgende Addition des Alkohols ROH, ferner Dodecatetraen und Dialkoxybutan. Die Bildung dieser Nebenprodukte kann durch die Art und Weise der Reaktionsführung in der Teilreaktion (a), beispielsweise durch die Wahl des 1,3-Butadien/ Alkohol ROH-Verhältnisses, d.h. einem Überschuß an Butanol, in der Reaktionsmischung, die Wahl der Reaktionstemperatur und des Drucks beeinflußt und gewünschtenfalls minimiert werden.

Das zur Herstellung von n-Butylalkylethern benötigte Addukt der Formel II, wird von seinem im Reaktionsaustrag größenordnungsmäßig in der gleichen Menge enthaltenen Isomeren 3-Alkoxybuten-1 der Formel III in der Destillationsstufe (b) abgetrennt und das unerwünschte Isomere 3-Alkoxybuten-1 III in das gewünschte 1-Alkoxybuten-2 II umgewandelt.

Die Abtrennung von Addukt III kann vorteilhaft so geschehen, daß der Reaktionsaustrag aus Teilreaktion (a), nach vorheriger Abtrennung nicht umgesetzten 1,3-Butadiens, z.B in einem Gas-Flüssigkeit-Abscheider oder einer Druckdestillation durch fraktionierte Destillation abgetrennt wird.

Bei dieser fraktionierten Destillation können auch die im Reaktionsaustrag von Teilreaktion a) enthaltenen Nebenprodukte, 1,3-Butadien-Dimere und -Trimere sowie deren Addukte mit dem Alkohol ROH I und gegebenenfalls mehrfach alkoxylierte Nebenprodukte sowie überschüssiger Alkohol ROH I vom Addukt II abgetrennt werden. Da diese Nebenprodukte sich im weiteren Gang des Verfahrens im allgemeinen nicht störend auswirken, kann deren Abtrennung auch unterbleiben und die Rückgewinnung erfolgt erst mit Destillation der Alkylbutylether. Es kann bei der Destillation auch so verfahren werden, daß man außer dem Addukt III nur einen Teil der Nebenprodukte, insbesondere die olefinischen 1,3-Butadien-Dimere und -Trimere sowie mehrfach alkoxylierte Nebenprodukte, abtrennt, hingegen andere Nebenprodukte, insbesondere das Alkoxyoctadien und gewünschtenfalls das Alkoxydodecatrien, gemeinsam mit dem Addukt II in den nachfolgenden Teilreaktionen weiterverarbeitet, wobei aus diesen Nebenprodukten der Teilreaktion (a) als Endprodukte Octyl- bzw. Dodecylalkylether entstehen.

Nach der bevorzugten Ausführungsform des erfindungsgemäßen Verfahrens wird das vom gewünschten Addukt abgetrennte Addukt III ebenso wie das nicht umgesetzte 1,3-Butadien in die Verfahrensstufe der Teilreaktion (a) zurückgeführt, wo die Isomerisierung des Addukts III zum Addukt II gleichzeitig mit der Additionsreaktion stattfindet bzw. die Rückführung zur Unterdrückung der Neubildung des unerwünschten Addukts III führt, so daß bei Anwendung dieser Kreislauffahrweise in der Gesamtbilanz praktisch nur das erwünschte Addukt II, nicht jedoch dessen unerwünschtes Isomer III gebildet wird.

Die Isomerisierung des Addukts III kann statt durch dessen Rückführung in die Verfahrensstufe der Teilreaktion (a) auch in einer separaten Isomerisierungsstufe (c) bewerkstelligt werden, indem man das vom Addukt II abgetrennte Addukt III z.B. durch einen mit einem Isomerisierungskatalysator, d.i. in der Regel ein wie in Stufe (a) verwendeter Katalysator, beschickten Reaktor leitet, den Austrag dieses Reaktors, der aus dem darin gebildeten Isomerisierungsgemisch aus Addukt III und Addukt II besteht, beispielsweise destillativ in Addukt II und Addukt III auftrennt, das neu gebildete Addukt II zu den n-Butylalkylethern weiterverarbeitet und das Addukt III wieder in den Isomerisierungsreaktor zurückführt.

Die Isomerisierung des Addukts III zu Addukt II kann in An- oder Abwesenheit eines Lösungsmittels erfolgen; vorzugsweise wird ohne Lösungsmittel gearbeitet. Wird die Isomerisierung in Gegenwart eines Lösungsmittels durchgeführt, werden im allgemeinen hochsiedende Lösungsmittel wie Ether, beispielsweise Di- oder Triethylenglykoldimethylether, Di- oder Triethylenglykoldibutylether, Sulfoxide, z.B. Dimethylsulfoxid oder Sulfone, wie Sulfolan, hochsiedende aromatische oder aliphatische Kohlenwasserstoffe oder halogenierte aliphatische oder aromatische Lösungsmittel, z.B. Dichlorbenzol, eingesetzt. Die Verwendung niedrig siedender Lösungsmittel ist ebenfalls möglich, erfordert in der Regel aber einen erhöhten Aufwand bei der destillativen Auftrennung des Austrags aus dem Isomerisierungsreaktor in die Addukte II und III.

Auch die Isomerisierung der 3-Alkoxybut-1-ene zu den 1-Alkoxybut-2-enen erfolgt generell nach den Angaben der WO 95/19334, so daß bezüglich der Katalysatoren und der Verfahrensführung ausdrücklich auf diese Referenz Bezug genommen wird und die dort gemachten Angaben als hier inkorporiert gelten sollen.

Nach einer bevorzugten Ausführungsform wird die Addition der Stufe (a) und die Isomerisierung (c) in Gegenwart von bis zu 20 Gew.-%, z.B. 0,0001 bis 20 Gew.-%, vorzugsweise 0,001 bis 10 Gew.-% und insbesondere 0,01 bis 5 Gew.-% Wasser durchgeführt.

Durch diese Maßnahme erhöht sich die Ausbeute an den gewünschten Alkylbutenylethern.

Das Wasser kann durch Zusatz zu irgendeiner der Stufen (a) bis (c) oder durch Unterlassung der Entfernung von Wasser aus dem Ionenaustauscher - wie dies sonst üblich ist - eingebracht werden. Bei der Trennung der Isomeren II und III durch Destillation bilden sich in der Regel Azeotrope aus Ether, Alkohol und Wasser, so daß bei Rückführung des Azeotrops, das den 3-Alkylbutenylether enthält, ein bestimmter Wassergehalt in der Stufe 1 gewährleistet ist. Dieser Wassergehalt reicht in der Regel aus. Gegebenenfalls kann der Wassergehalt durch weiteren Wasserzusatz weiter erhöht werden. Der optimale Wassergehalt wird zweckmäßig je nach Art des Katalysators durch einen Vorversuch ermittelt. Er beträgt z.B. bei Verwendung von PUROLITE® CT 175 (Fa. Purolite, Deutschland GmbH Ratingen) 0,5 bis 2 Gew.-%.

In einer vierten Stufe (d) wird das Addukt II katalytisch unter Verwendung an sich bekannter Katalysatoren zum Ether der Formel IV hydriert.

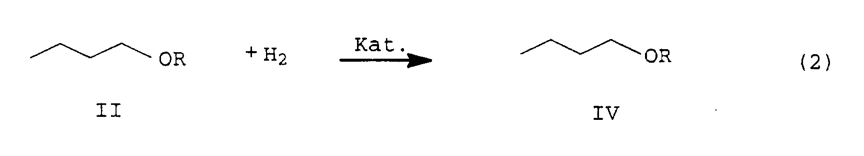

Die Hydrierung des Addukts II zum n-Butylalkylether IV kann bevorzugt in der Gasphase oder auch in flüssiger Phase ausgeführt werden. Bei der Durchführung des Reaktionsschrittes in flüssiger Phase können sowohl homogene als auch heterogene Katalysatoren angewandt werden. Wird in der Gasphase gearbeitet, werden im allgemeinen heterogene Katalysatoren bevorzugt.

Als Homogenkatalysatoren zur Hydrierung des Addukts II zum n-Butylalkylether IV können eine Vielzahl von Übergangsmetallelementverbindungen eingesetzt werden, insbesondere solche, die Elemente aus der V., VI., VII. und VIII. Nebengruppe des Periodensystems der Elemente, vorzugsweise Eisen, Kobalt, Nickel, Ruthenium, Rhodium, Palladium, Platin, Osmium und/oder Iridium enthalten.

Als Katalysatoren eignen sich beispielsweise die Salze dieser Übergangsmetalle, insbesondere deren im Reaktionsmedium löslichen Halogenide, Nitrate, Sulfate, Phosphate, Carboxylate, beispielsweise deren C₁- bis C₂₀-Carboxylate, wie Formiate, Acetate, Trichloracetate, Propionate, 2-Ethylhexanoate, Dekanoate, ferner die Citrate, Tartrate, Malate, Malonate, Maleinate oder Fumarate, Sulfonate, beispielsweise Methansulfonate, Benzolsulfonate, Naphthalinsulfonate, Toluolsulfonate oder Trifluormethansulfonate, Cyanide, Tetrafluoroborate, Perchlorate oder Hexafluorophosphate, weiterhin lösliche Salze der Oxosäuren dieser Metalle, insbesondere die Alkalimetall-, Erdalkalimetall- oder Oniumsalze, wie Ammonium-, Phosphonium-, Arsonium- oder Stiboniumsalze, lösliche anorganische Komplexverbindungen dieser Elemente, insbesondere deren Aquo-, Halogeno-, Phosphin-, Phosphit-, Cyano- oder Amino-Komplexe sowie die Komplexe dieser Übergangsmetalle mit Chelatbildnern, wie Acetylaceton, Dioximen, beispielsweise Diacetyldioxim, Furildioxim oder Benzildioxim, Ethylendiamintetraessigsäure, Nitrilotriessigsäure, Nitrilotriethanol, Harnstoffen oder Thioharnstoffen, Bisphosphinen, Bisphosphiten, Bipyridinen, Terpyridinen, Phenanthrolinen, 8-Hydroxychinolin, Kronenethern oder Polyalkylenglykolen, sowie Organometallverbindungen dieser Übergangsmetallelemente, beispielsweise Carbonylkomplexe wie HRuCl (CO) (PPh₃)₃, HRuCl (CO) (Hexyldiphenylphosphin)₃, RuH₂(CO)(PPh₃)₃, RuH (CO) (CH₃CO₂)(PPh₃)₂, RuH₂(PPh₃)₃, HRh(CO)(PPh₃)₃, oder IrCl(CO)(PPh₃ = Triphenylphosphin).

Bevorzugte salzartige Homogenkatalysatoren sind die Halogenide, insbesondere die Chloride, Nitrate, Sulfate, Sulfonate, Carboxylate und Cyanide des Rhodiums, Rutheniums, Palladiums, Platins und Iridiums.

Bevorzugte Homogenkatalysatoren für die Durchführung der Hydrierung (d) sind Komplexe der genannten Übergangsmetallelemente, insbesondere des Kobalts, Nickels, Rhodiums, Rutheniums, Palladiums, Platins und Iridiums mit einzähnigen oder mehrzähnigen, insbesondere zweizähnigen, Phosphin- oder Phosphit-Liganden und/oder mit stickstoffhaltigen Liganden, für deren Eigenschaft als Komplexbildner die (-N=C-C=N-)-Struktureinheit ursächlich ist, beispielsweise 2,2'-Bipyridin oder 1,10-Phenanthrolin, sowie die durch Substitution oder Anellierung von diesen Grundkörpern abgeleiteten Liganden.

Übergangsorganometallelement-Verbindungen, die besonders bevorzugt als Homogenkatalysatoren zur Durchführung der Teilreaktion (d) verwendet werden, sind z.B. Carbonylkomplexe, wie HRh(PPh₃)₃(CO), HRUCl(CO) (PPh₃)₃, RuH(CO) (CH₃CO₂) (PPh₃)₂, RuH(CO) (C₉H₁₉CO₂) (PPh₃)₂ oder RuCl₂(CO)₂(PPh₃)₃.

Geeignete Phosphin-Liganden sind beispielsweise die gleichen der zur Durchführung der Teilreaktion (a) genannten Phosphin-Liganden, auf die hiermit verwiesen wird. Geeignete 2,2'-Bipyridin- oder 1,10-Phenanthrolin-Liganden sind beispielsweise die zur Durchführung der Teilreaktion (a) genannten Liganden sowie deren am angegebenen Ort genannten Derivate und Strukturanaloge, auf die hiermit verwiesen wird.

Geeignete Phosphit-Liganden sind z.B. Trialkylphosphite, Alkyldiarylphosphite, Triarylphosphite, Alkyl-bisphosphite, Aryl-bis-phosphite, Alkylaryl-bisphosphite. Die Alkylgruppen-tragenden Phosphit-Liganden können gleiche oder verschiedene C₁- bis C₁₀-, vorzugsweise C₁- bis C₆-Alkyl- oder Cycloalkylgruppen enthalten. Die Arylgruppen-tragenden Phosphit-Liganden können gleiche oder verschiedene C₆- bis C₁₂-Arylgruppen, insbesondere die Phenyl- oder Naphthylgruppe, aber auch die Diphenylgruppe enthalten. Weiterhin können Phosphit-Liganden zur Komplexierung der Übergangsmetalle eingesetzt werden, die heterocycloaliphatische Gruppen, wie Pyrrolidin-, Imidazolidin-, Piperidin-, Morpholin-, Oxazolidin-, Piperazin- oder Triazolidin-Gruppen oder heteroaromatische Gruppen, wie Pyrrol-, Imidazol-, Oxazol-, Indol-, Pyridin-, Chinolin-, Pyrimidin-, Pyrazol-, Pyrazin-, Pyridazin- oder Chinoxazolin-Gruppen gemeinsam mit anderen Alkyl- oder Aryl-Gruppen tragen. Die Alkyl- oder Arylgruppen der Phosphit-Liganden können unsubstituiert sein oder unter den Reaktionsbedingungen inerte Substituenten, wie C₁- bis C₄-Alkoxy-, Di-C₁- bis C₄-Alkylamino-, C₁- bis C₆-Alkyl- oder Hydroxy-, Nitro-, Cyano- oder Sulfonat-Gruppen tragen. Die sulfonatsubstituierten Phosphit-Liganden und deren Komplexe sind im allgemeinen wasserlöslich. Geeignete Phosphit-Liganden sind z.B. Trimethylphosphit, Triethylphosphit, Tripropylphosphit, Triisopropylphosphit, Tributylphosphit, Tricyclopentylphosphit, Tricyclohexylphosphit, Triphenylphosphit sowie die in EP-A 472 071, EP-A 213 639, EP-A 214 622, DE-A 27 33 796, EP-A 2 261, EP-A 2 821, EP-A 9 115, EP-A 155 508, EP-A 353 770, US-A 4 318 845, US-A 4 204 997 und US-A 4 362 830 beschriebenen Mono- und Bisphosphit-Liganden.

Bei der Hydrierung (d) mit homogen im Reaktionsmedium löslichen Phosphin- oder Phosphit-Komplexen als Katalysatoren kann es sich als vorteilhaft erweisen, zusätzlich ein Phosphin oder Phosphit, vorzugsweise das im eingesetzten Homogenkatalysator als Ligand dienende Phosphin oder Phosphit, und/oder eine Säure, wie Alkansäuren z.B. Essigsäure, Ethylhexansäure oder Dekansäure oder Toluolsulfonsäure oder Phenole zur Reaktionsmischung zu zugeben. Ein solcher Zusatz kann eine Verlängerung der Standzeit des Homogenkatalysators bewirken und darüber hinaus die Selektivität der Umsetzung des Addukts II zum n-Butylalkylether IV und somit die Selektivität des Gesamtverfahrens verbessern. Einen ähnlichen vorteilhaften Effekt kann der Zusatz von Kohlenmonoxid zum Reaktionsgemisch auslösen, insbesondere bei Verwendung carbonylgruppenhaltiger Übergangsmetallelementkomplexe als Homogenkatalysatoren.

Zur Erzielung der obengenannten Wirkungen wird im allgemeinen das Phosphin oder Phosphit bezüglich des Phosphin- oder Phosphit-Komplexes des Übergangsmetallelementes in einer 1 bis 100 molaren, vorzugsweise in einer 1 bis 20 molaren und besonders bevorzugt in einer 1 bis 5 molaren Menge zugesetzt. Wird der als Homogenkatalysator dienende Übergangsmetallelementkomplex in situ im Reaktionsgemisch erzeugt, so setzt man zweckmäßigerweise einen entsprechend hohen Überschuß an Phosphin- oder Phosphit-Ligand bezüglich des betreffenden Übergangsmetallelementes ein.

Die homogen im Reaktionsmedium löslichen Übergangsmetallkatalysatoren werden im allgemeinen bezogen auf das dem Reaktor zugeführte Addukt II in Mengen von vorzugsweise 0,02 bis 0,2 mol-% eingesetzt. Zweckmäßigerweise wird die optimale Menge für den jeweils verwendeten Homogenkatalysator in einem Vorversuch ermittelt.

Die Hydrierung (d) mit Hilfe der genannten Homogenkatalysatoren kann diskontinuierlich, z.B. in Rührkesseln oder kontinuierlich, z.B. in Schlaufenreaktoren, Blasensäulen oder Rührkesselkaskaden, bei Temperaturen von im allgemeinen mehr als 60°C und bei einem Druck von im allgemeinen 5 bis 100 bar, vorzugsweise von 10 bis 60 bar vorgenommen werden. Die Umsetzung des Addukts II zum n-Butylalkylether IV kann in An- oder Abwesenheit von zugesetzten Lösungsmitteln, wie aliphatischen oder aromatischen Kohlenwasserstoffen, z.B. Toluol, Benzol, Mihagol oder Cyclohexan, Alkoholen, z.B. Butanole, höhere Fettalkohole oder Glykole, Ethern, z.B. Tetrahydrofuran, Dioxan oder niedermolekulare Polyalkylenglykole, halogenierten aliphatischen oder aromatischen Kohlenwasserstoffen, z.B. Chloroform, Dichlormethan, Chlorbenzol, Dichlorbenzol, Sulfoxiden oder Sulfonen, z.B. Dimethylsulfoxid oder Sulfolan erfolgen.

Werden bei der Hydrierung keine weiteren Lösungsmittel zugesetzt, so bewirken auch die Reaktanten selbst, also das Addukt II und der n-Butylalkylether IV die Lösung der Homogenkatalysatoren.

Zur Hydrierung des Addukts II wird Wasserstoff in einem Molverhältnis, bezogen auf das dem Reaktor zugeführte Addukt II, von im allgemeinen 1:1 bis 100:1, vorzugsweise von 1:1 bis 50:1 und besonders bevorzugt von 1:1 bis 10:1 zugemischt. Diese Zumischung kann bei diskontinuierlicher Betriebsweise des Verfahrens durch Einpressen der erforderlichen Wasserstoffmenge in den Reaktor oder durch Dispergieren des Wasserstoffs im Reaktionsmedium, beispielsweise mittels Blasensäulen oder mittels Strahldüsen zur Dispergierung des Wasserstoffs ausgestatteten Schlaufenreaktoren erfolgen. Die Zumischung des Wasserstoffs erfolgt in der Regel bei der Beschickung des Reaktors mit dem Addukt II und dem Homogenkatalysator, kann aber auch vorteilhaft nachträglich in die Reaktionsapparatur eingespeist werden. Welche dieser Verfahrensweisen gewählt wird, hängt vom verwendeten Katalysator und den jeweils angewandten Druck- und Temperaturbedingungen sowie von der Konstruktion des Reaktors ab. Zweckmäßigerweise wird die optimale Verfahrensweise in einem Vorversuch ermittelt. In analoger Weise kann bei der kontinuierlichen Ausgestaltung der Hydrierung, z.B. in einem Rohrreaktor, einem Blasensäulenreaktor oder einer Füllkörperkolonne, der Wasserstoff gemeinsam mit den übrigen Reaktanten oder getrennt kontinuierlich in den Reaktor eingebracht werden.

Nach beendeter Umsetzung wird das Reaktionsprodukt im allgemeinen destillativ aufgearbeitet, wobei der verwendete Homogenkatalysator aus dem Sumpf der Destillation zurückgewonnen und gewünschtenfalls erneut verwendet werden kann. Ist die Rückführung des Katalysators im erfindungsgemäßen Verfahren erwünscht, kann dem Reaktionsgemisch zweckmäßigerweise noch ein Lösungsmittel, vorzugsweise ein Lösungsmittel, das bei einer höheren Temperatur als die n-Butylalkylether siedet, zugefügt werden. Ist der verwendete Homogenkatalysator unter den Bedingungen der Destillation chemisch und thermisch stabil, kann vom Zusatz eines hochsiedenden Lösungsmittels abgesehen und der Homogenkatalysator in fester Form wieder in die Umsetzung zurückgeführt werden.

In einer weiteren bevorzugten Ausgestaltung des erfindungsgemäßen Verfahrens wird die Hydrierung des Addukts II zum n-Butylalkylether IV unter Verwendung eines heterogenen Katalysators ausgeführt, wobei das Verfahren wahlweise in flüssiger Phase oder vorzugsweise in der Gasphase durchgeführt wird.

Von diesen Hydrierkatalysatoren sind solche bevorzugt, die ein oder mehrere Elemente der Gruppe Ib, VIb, VIIb und VIIIb, gegebenenfalls in Kombination mit einem oder mehreren Elementen der Gruppe Vb, des Periodensystems der Elemente, insbesondere Kupfer, Zink, Chrom, Molybdän, Wolfram, Rhenium, Ruthenium, Kobalt, Nickel, Rhodium, Iridium, Palladium und/oder Platin, gegebenenfalls in Kombination mit Eisen, enthalten.

Besonders aktive Hydrierkatalysatoren wie Nickel, Kobalt oder die Platinmetalle können vorteilhaft mit Hauptgruppenelementen, die als Katalysatorgift wirken, dotiert und auf diese Weise partiell vergiftet werden. Durch diese Maßnahme kann eine höhere Selektivität bei der Hydrierung zu den n-Butylalkylethern erzielt werden. Geeignete Hauptgruppenelemente sind z.B. die Chalcogene, wie Schwefel, Selen und Tellur, sowie die Elemente Phosphor, Arsen, Antimon, Wismut, Zinn, Blei. und Thallium.

Als Heterogenkatalysatoren können z.B. sogenannte Fällungskatalysatoren verwendet werden. Solche Katalysatoren werden hergestellt, indem man ihre katalytisch aktiven Komponenten aus deren Salzlösungen, insbesondere aus den Lösungen von deren Nitraten und/oder Acetaten, beispielsweise durch Zugabe von Lösungen von Alkalimetall- und/oder Erdalkalimetallhydroxid und/oder Carbonat-Lösungen, als z.B. schwerlösliche Hydroxyde, Oxidhydrate, basische Salze oder Carbonate ausfällt, die erhaltenen Niederschläge anschließend trocknet und diese dann durch Calcinierung bei im allgemeinen 300 bis 700°C, insbesondere 400 bis 600°C in die betreffenden Oxide, Mischoxide und/oder gemischtvalentigen Oxide umwandelt, welche z.B. durch eine Behandlung mit Reduktionsmitteln, wie Wasserstoff oder Wasserstoff enthaltenden Gasen, bei in der Regel 50 bis 700°C, insbesondere bei 100 bis 400°C, zu den betreffenden Metallen und/oder zu oxidischen Verbindungen niedriger Oxidationsstufe reduziert und in die eigentliche, katalytisch aktive Form überführt werden. Dabei wird in der Regel solange reduziert, bis kein Wasser mehr gebildet wird. Bei der Herstellung von Fällungskatalysatoren, die ein Trägermaterial enthalten, kann die Fällung der katalytisch aktiven Komponenten in Gegenwart des betreffenden Trägermaterials erfolgen. Die katalytisch aktiven Komponenten können vorteilhaft aber auch gleichzeitig mit dem Trägermaterial aus den betreffenden Salzlösungen gefällt werden.

Bevorzugt werden Hydrierkatalysatoren eingesetzt, welche die Hydrierung katalysierenden Metalle oder Metallverbindungen auf einem Trägermaterial abgeschieden enthalten. Außer den obengenannten Fällungskatalysatoren, welche außer den katalytisch aktiven Komponenten noch zusätzlich ein Trägermaterial enthalten, eignen sich für die Hydrierung (d) im allgemeinen solche Trägerkatalysatoren, bei denen die katalytisch wirkenden Komponenten z.B. durch Imprägnierung auf ein Trägermaterial aufgebracht worden sind.

Die Art der Aufbringung der katalytisch aktiven Metalle auf den Träger ist in der Regel nicht kritisch und kann auf verschiedenerlei Art und Weise bewerkstelligt werden. Die katalytisch aktiven Metalle können auf diese Trägermaterialien z.B. durch Tränkung mit Lösungen oder Suspensionen der Salze oder Oxide betreffenden Elemente, Trocknung und anschließende Reduktion der Metallverbindungen zu den betreffenden Metallen oder Verbindungen niederer Oxidationsstufe mittels eines Reduktionsmittels, vorzugsweise mit Hilfe von Wasserstoff, Wasserstoff enthaltenden Gasen oder Hydrazin, aufgebracht werden. Eine andere Möglichkeit zur Aufbringung der katalytisch aktiven Metalle auf diese Träger besteht darin, die Träger mit Lösungen thermisch leicht zersetzbarer Salze, z.B. mit Nitraten oder mit thermisch leicht zersetzbaren Komplexverbindungen, z.B. Carbonyl- oder Hydrido-Komplexen der katalytisch aktiven Metalle, zu imprägnieren und den so getränkten Träger zwecks thermischer Zersetzung der adsorbierten Metallverbindungen auf Temperaturen von 300 bis 600°C zu erhitzen. Diese thermische Zersetzung wird vorzugsweise unter einer Schutzgasatmosphäre vorgenommen. Geeignete Schutzgase sind z.B. Stickstoff, Kohlendioxid, Wasserstoff oder die Edelgase. Weiterhin können die katalytisch aktiven Metalle auf dem Katalysatorträger durch Aufdampfung oder durch Flammspritzen abgeschieden werden.

Der Gehalt dieser Trägerkatalysatoren an den katalytisch aktiven Metallen ist prinzipiell für das Gelingen des erfindungsgemäßen Verfahrens nicht kritisch, wobei höhere Gehalte dieser Trägerkatalysatoren an katalytisch aktiven Metallen natürlich in der Regel zu höheren Raum-Zeit-Umsätzen führen als niedrigere Gehalte. Im allgemeinen werden aber Trägerkatalysatoren verwendet, deren Gehalt an katalytisch aktiven Metallen 0,1 bis 80 Gew.-%, vorzugsweise 0,5 bis 50 Gew.-%, bezogen auf den gesamten Katalysator beträgt. Selbstverständlich können auch mehrere der katalytisch aktiven Metalle auf dem jeweiligen Trägermaterial aufgebracht sein. Weiterhin können die katalytisch aktiven Metalle beispielsweise nach den Verfahren von DE-A 2519817, EP-A 147219 und EP-A 285420 auf den Träger aufgebracht werden. In den Katalysatoren gemäß den vorgenannten Schriften liegen die katalytisch aktiven Metalle als Legierung vor, die durch thermische Behandlung und/oder Reduktion der z.B. durch Tränkung auf einem Träger abgeschiedenen Salze oder Komplexe der zuvor genannten Metalle, erzeugt werden.

Die Aktivierung der Fällungskatalysatoren als auch der Trägerkatalysatoren kann auch in situ in der Reaktionsmischung durch den dort anwesenden Wasserstoff erfolgen, bevorzugt werden diese Katalysatoren jedoch vor ihrer Verwendung aktiviert.

Als Trägermaterialien können im allgemeinen die Oxide des Aluminiums und Titans, Zirkoniumdioxid, Siliziumdioxid, Kieselgur, Kieselgel, Tonerden, z.B. Montmorillonite, Silikate, wie Magnesium- oder Aluminiumsilikate, Zeolithe, wie ZSM-5 oder ZSM-10-Zeolithe sowie Aktivkohle verwendet werden. Bevorzugte Trägermaterialien sind Aluminiumoxide, Titandioxide, Zirkoniumdioxid und Aktivkohle.

Als in Betracht kommende Heterogen-katalysatoren seien die folgenden Katalysatoren beispielhaft genannt: Platindioxid, Palladium auf Aluminiumoxid, Palladium auf Silizium-dioxid, Palladium auf Bariumsulfat, Rhodium auf Aktivkohle, Rhodium auf Aluminiumoxid, Ruthenium auf Siliziumdioxid oder Aktivkohle, Nickel auf Siliziumdioxid, Nickel auf Aluminiumoxid, Nickel auf Zirkoniumdioxid, Kobalt auf Siliziumdioxid, Kobalt auf Aluminiumoxid, Kobalt/Molybdän auf Aluminiumoxid, Carbonyleisenpulver, Rhenium-schwarz, Raney-Rhenium, Rhenium auf Aktivkohle, Rhenium-Palladium auf Aktivkohle, Rhenium-Platin auf Aktivkohle, Kupfer auf Siliziumdioxid, Kupfer auf Aluminiumoxid, Kupfer auf Aktivkohle, Kupfer auf Kieselgur, Kupfer auf Kieselgel, Kupfer auf Titandioxid, Kupfer auf Zirkoniumdioxid, Kupfer auf Magnesiumsilikat, Kupfer auf Aluminiumsilikat, Kupfer auf Montmorillonit, Kupfer auf Zeolith, Kupfer/Zink auf Aluminiumoxid, Raney-Kupfer, Platinoxid-Rhodiumoxid-Mischungen, Platin-Palladium auf Aktivkohle, Kupferchromit, Bariumchromit, Nickel-Chromoxid auf Aluminiumoxid, Raney-Nickel, Kobaltsulfid, Nickelsulfid, Molybdän(VI)sulfid, Kupfer-Molybdän(VI)oxid-Siliziumdioxid-Aluminiumoxid-Katalysatoren, mit Selen oder Blei partiell vergiftete Palladium auf Aktivkohle-Katalysatoren sowie die Katalysatoren gemäß DE-A 39 32 332, US-A 3 449 445, EP-A 44 444, EP-A 147 219, DE-A 39 04 083, DE-A 23 21 101, EP-A 415 202, DE-A 23 66 264 und EP-A 100 406.

Vorteilhaft können im erfindungsgemäßen Verfahren auch Hydrierkatalysatoren verwendet werden, die Brönsted- und/oder Lewisacide- oder basische Zentren enthalten. Bei der Verwendung derartiger Katalysatoren ist im allgemeinen die zusätzliche Zugabe einer Brönsted- oder Lewis-Säure oder Basen zur Reaktionsmischung nicht erforderlich.

Als Brönsted- oder Lewis-saure Zentren können z.B. die katalytisch aktiven Metalle selber wirken, wenn diese bei der Aktivierung des Katalysators mit Wasserstoff oder Wasserstoff enthaltenden Gasen nicht vollständig zu den betreffenden Metallen reduziert werden. Dies gilt z.B. für die rhenium- und chromithaltigen Katalysatoren, wie Rheniumträgerkatalysatoren und Kupferchromit. In den Rheniumträgerkatalysatoren liegt das Rhenium als Mischung von Rheniummetall mit Rhenium-verbindungen in höheren Oxidationsstufen vor, wobei letztere Wirkungen wie Lewis- oder Brönsted-Säuren entfalten können. Weiterhin können solche Lewis- oder Brönsted-aciden oder basiche Zentren über das verwendete Trägermaterial in den Katalysator eingebracht werden. Als Lewis- oder Brönsted-acide Zentren enthaltende Trägermaterialien seien z.B. die Aluminiumoxide, Titandioxide, Zirkoniumdioxid, Siliziumdioxid, die Silikate, Tonerden, Zeolithe und Aktivkohle genannt.

Bevorzugt werden deshalb im erfindungsgemäßen Verfahren als Hydrierkatalysatoren Trägerkatalysatoren verwendet, die Elemente der I., VI., VII. und/oder VIII. Nebengruppe des Periodensystems der Elemente, insbesondere der Elemente der I., VII. und VIII. Nebengruppe des Periodensystems der Elemente auf einem Brönsted- oder Lewis-acide wirkenden Trägermaterial abgeschieden enthalten. Besonders vorteilhafte Katalysatoren sind z.B. Kupfer auf Aktivkohle, Kupfer auf Siliziumdioxid, Kupfer auf Kieselgur, Kupfer auf Kieselgel, Kupfer auf Titandioxid, Kupfer auf Zirkoniumdioxid, Kupfer auf Magnesiumsilikat, Kupfer auf Aluminiumsilikat, Kupfer auf Bleicherde, Kupfer auf Zeolith, Ruthenium auf Aktivkohle, Ruthenium auf Aluminiumoxid, Ruthenium auf Siliziumdioxid, Ruthenium auf Magnesiumoxid, Ruthenium auf Zirkoniumdioxid, Ruthenium auf Titandioxid, Palladium auf Aluminiumoxid, Palladium auf Siliziumdioxid, Palladium auf Bariumsulfat und mit Selen oder Blei partiell vergiftete Palladium auf Aktivkohle-Katalysatoren, Platin auf Aluminiumoxid, Platin auf Siliziumdioxid, Nickel auf Zirkoniumdioxid, Nickel auf Siliziumdioxid, Nickel auf Aluminiumoxid, Nickel-Kupfer auf Aluminiumoxid, Kobalt auf Siliziumdioxid, Kobalt auf Aluminiumoxid, Kobalt-Molybdän auf Aluminiumoxid.

Hydrierkatalysatoren, welche selbst keine derartigen Brönsted- oder Lewis-aciden Zentren haben, können Lewis- oder Brönsted-acide-Komponenten, wie Zeolithe, Aluminium- oder Siliziumoxide, Phosphorsäure oder Schwefelsäure, zugesetzt werden. Sie werden im allgemeinen in Mengen von 0,01 bis 5 Gew.-%, vorzugsweise von 0,05 bis 0,5 Gew.-% und besonders bevorzugt von 0,1 bis 0,4 Gew.-%, bezogen auf das Gewicht des eingesetzten Katalysators, zugesetzt.

Weiterhin sind zur Hydrierung des Addukts II zum n-Butylalkylether IV Heterogenkatalysatoren geeignet, welche die zur homogenen Katalyse dieser Verfahrensstufe verwendbaren Komplexverbindungen von Übergangsmetallelementen aus der Gruppe VIb, VIIb und VIIIb des Periodensystems der Elemente in heterogenisierter Form enthalten, beispielsweise solche, in denen das betreffende Übergangsmetallelement an eine polymere Matrix fixiert ist.

Solche polymeren Matrizes können Harze, wie Styrol-Divinylbenzol-Harze oder Phenol-Formaldehyd-Harze sein, an die die betreffenden zur Komplexierung des Übergangsmetallelements dienenden Liganden vorzugsweise kovalent gebunden sind, welche wiederum mit den betreffenden Übergangsmetallen Komplexe bilden und diese derart quasi immobilisieren.

Mit den genannten Heterogenkatalysatoren kann die Hydrierung des Addukts II zum n-Butylalkylether IV sowohl kontinuierlich als auch diskontinuierlich durchgeführt werden.

Wird diese Umsetzung in flüssiger Phase durchgeführt, so kann der Heterogenkatalysator sowohl im flüssigen Reaktionsmedium suspendiert oder vorzugsweise in einem Festbett oder mehreren Festbetten angeordnet, eingesetzt werden. Das Verfahren kann bei Einsatz eines im flüssigen Reaktionsmedium suspendierten Heterogenkatalysators z.B. in Rührkesseln oder Schlaufenreaktoren durchgeführt werden. Bei Anwendung eines in einem Festbett angeordneten Heterogenkatalysators wird das Reaktionsgut im allgemeinen in der Sumpf- oder Rieselfahrweise über das Katalysatorfestbett geleitet.

Die Hydrierung kann in adiabatisch oder isotherm betriebenen Reaktoren vorgenommen werden. Im allgemeinen wird hierbei der Katalysator mit der flüssigen Reaktionsmischung mit einer Raumgeschwindigkeit von 0,01 bis 10, vorzugsweise von 0,05 bis 6 und besonders bevorzugt von 0,08 bis 3 kg Reaktionsgemisch/l Katalysator · h belastet. Bei Anwendung der Heterogenkatalysatoren kann die Umsetzung in An- oder Abwesenheit eines Lösungsmittels erfolgen. Als Lösungsmittel können die gleichen Lösungsmittel Anwendung finden, welche auch bei der Durchführung des Verfahrens unter homogener Katalyse verwendet werden können.

Der Wasserstoff wird bei heterogener Katalyse, bezogen auf das dem Reaktor zugeführte Addukt II, in einem Molverhältnis von im allgemeinen 1 bis 100, vorzugsweise von 1,5 bis 80, insbesondere von 2 bis 40 zugesetzt. Die Hydrierung des Addukts II zum n-Butylalkylether IV am Heterogenkatalysator in flüssiger Phase wird im allgemeinen bei einer Temperatur von 20 bis 400°C, vorzugsweise von 30 bis 350°C und besonders bevorzugt von 80 bis 250°C und bei einem Druck von im allgemeinen 1 bis 300 bar, vorzugsweise von 5 bis 250 bar, insbesondere von 20 bis 200 bar ausgeführt.

Der flüssige Reaktionsaustrag aus Stufe (d) wird im allgemeinen destillativ, wie es für die Homogenkatalysatoren bereits beschrieben wurde, aufgearbeitet.

Die Hydrierung kann auch in der Gasphase durchgeführt werden. Hierzu können an sich übliche Reaktoren für Gasphasenreaktionen verwendet werden, beispielsweise solche, in denen der Katalysator in einem Festbett oder in einer Wirbelschicht angeordnet ist. Die Reaktoren können adiabatisch oder isotherm betrieben werden. Bei Anwendung einer Festbettanordnung des Katalysators kann der Katalysator in einem oder vorteilhaft zwecks Verbesserung der Abführung der Reaktionswärme in mehreren, beispielsweise 2 bis 10, vorzugsweise 2 bis 5 Festbetten, angeordnet sein. Bei Verwendung mehrerer Katalysatorfestbette oder bei adiabatischer Betriebsweise des Reaktors kann es vorteilhaft sein, durch eine Zwischenbettkühlung des Reaktionsgases und/oder durch Eindüsen zusätzlicher Mengen an kühlen Reaktanten wie Wasserstoff oder Addukt II zwischen den einzelnen Festbetten, die Temperatur des Reaktionsgases nach Verlassen des vorhergehenden Festbetts und vor Eintritt in das nachfolgende Festbett abzusenken, um die Selektivität der Umsetzung zu erhöhen. Zweckmäßigerweise wird bei Anwendung mehrerer Festbette in den einzelnen Festbetten vor dem letzten Festbett die Umsetzung nur bis zu einem Teilumsatz, beispielsweise bis zu einem Umsatz von 50 bis 98%, durchgeführt. Die Reaktionsgase können gewünschtenfalls mit einem unter den Reaktionsbedingungen inerten Gas, wie Stickstoff, gesättigten Kohlenwasserstoffen oder Argon, verdünnt werden.

Der Wasserstoff wird bei der Hydrierung in der Gasphase, bezogen auf das dem Reaktor zugeführten Addukt II, in einem Molverhältnis von im allgemeinen 1 bis 200, vorzugsweise von 1,5 bis 80 und besonders bevorzugt von 2 bis 40 zugesetzt. Wasserstoff kann dem Reaktor gemeinsam mit dem Addukt II und/oder in mehrere Teilströme aufgeteilt, an verschiedenen Stellen des Reaktors zugeführt werden. Im allgemeinen wird der Katalysator mit dem Reaktionsgas, enthaltend im wesentlichen das Addukt II, Wasserstoff und gewünschtenfalls ein Inertgas, mit einer Raumgeschwindigkeit von 0,01 bis 10, vorzugsweise von 0,05 bis 5, insbesondere von 0,07 bis 3 kg Reaktionsgas/1 Katalysator·h belastet. Die Umsetzung wird im allgemeinen bei Temperaturen von 20 bis 400°C, vorzugsweise von 100 bis 350°C und besonders bevorzugt von 150 bis 250°C und bei einem Druck von im allgemeinen 0,5 bis 100 bar, vorzugsweise von 0,9 bis 50 bar, insbesondere von 1 bis 20 bar vorgenommen.

Zur Aufarbeitung des gasförmigen Reaktionsaustrages kann dieser gegebenenfalls nach Entspannung auf Atmosphärendruck, direkt in eine Destillationsapparatur eingeleitet und dort destillativ in seine Bestandteile aufgetrennt werden.

Für die Hydrierung in der Gasphase können grundsätzlich die gleichen Heterogenkatalysatoren eingesetzt werden, wie sie auch bei der Umsetzung in flüssiger Phase Anwendung finden. Vorzugsweise werden im Gasphasenverfahren rein anorganische, mineralische Katalysatoren verwendet. Bevorzugte Katalysatoren sind beispielsweise Trägerkatalysatoren, die Elemente der I., VI., VII. und/ oder VIII. Nebengruppe, gegebenenfalls in Kombination mit einem oder mehreren Elementen der V. Nebengruppe, des Periodensystems der Elemente, insbesondere der Elemente der I., VII. und VIII. Nebengruppe des Periodensystems der Elemente auf einem Brönsted- oder Lewis-acide oder basisch wirkenden Trägermaterial abgeschieden enthalten. Vorteilhafte Katalysatoren sind z.B. Kupfer auf Aktivkohle, Kupfer auf Silizium-dioxid, Kupfer auf Kieselgur, Kupfer auf Kieselgel, Kupfer auf Titandioxid, Kupfer auf Zirkoniumdioxid, Kupfer auf Magnesiumsilikat, Kupfer auf Aluminiumsilikat, Kupfer auf Bleicherde, Kupfer auf Zeolith, Ruthenium auf Aktivkohle, Ruthenium auf Aluminiumoxid, Ruthenium auf Siliziumdioxid, Ruthenium auf Magnesiumoxid, Ruthenium auf Zirkoniumdioxid, Ruthenium auf Titandioxid, Palladium auf Aluminiumoxid, Palladium auf Siliziumdioxid, Palladium auf Bariumsulfat und mit Selen oder Blei partiell vergiftete Palladium auf Aktivkohle-Katalysatoren, Platin auf Aluminiumoxid, Platin auf Siliziumdioxid, Nickel auf Zirkoniumdioxid, Nickel auf Siliziumdioxid, Nickel auf Aluminiumoxid, Nickel-Kupfer auf Aluminiumoxid, Kobalt auf Siliziumdioxid, Kobalt auf Aluminiumoxid, Kobalt-Molybdän auf Aluminiumoxid.

Das erfindungsgemäße Verfahren wird anhand des Fließbilds gemäß Fig., das schematisch eine vorteilhafte Ausgestaltung wiedergibt, näher erläutert.

Über die Zuleitung 1 wird in den Reaktor 2 ein Gemisch aus 1,3-Butadien oder ein Butadien-haltiges Kohlenwasserstoffgemisch (3) und ein Alkohol ROH I (4), vorzugsweise n-Butanol, eingeleitet. Im Reaktor 2 wird der Alkohol ROH I katalytisch an 1,3-Butadien addiert, wobei sich ein Gemisch der Addukte II und III bildet. Der Reaktionsaustrag aus dem Reaktor 2, der im wesentlichen aus den Addukten II und III, höhersiedenden Butadien-Derivaten sowie nicht umgesetztem 1,3-Butadien und Alkohol ROH I besteht, wird über Leitung 5 dem Gas-Flüssigkeit-Abscheider 6 zugeführt, in dem gasförmiges 1,3-Butadien von den flüssigen Bestandteilen des Reaktionsaustrages abgetrennt und entweder über die Leitungen 7, 8 wieder in den Reaktor 2 zurückgeführt oder über die Leitung 9 abgefackelt wird. Das im Abscheider 6 anfallende Flüssigkeitsgemisch wird über Leitung 10 in die Destillationskolonne 11 geleitet, in der das leichter flüchtige Addukt III vom schwerer flüchtigen Addukt II sowie von noch gegebenenfalls vorhandenem Alkohol ROH I und höhersiedenden Butadien-Derivaten destillativ abgetrennt wird. Das Addukt III, nicht umgesetzter Alkohol ROH I sowie gegebenenfalls noch vorhandenes, nicht umgesetztes 1,3-Butadien werden anschließend über die Leitung 12 in den Reaktor 2 zurückgeleitet, wo das Addukt III in Gegenwart von frisch zugeführtem 1,3-Butadien und Alkohol ROH I zum Addukt II isomerisiert wird. Wahlweise kann auch der nicht umgesetzte Alkohol gegebenenfalls mit dem Addukt II über die Leitung 13 dem Reaktor 14 zugeführt werden. Die mit dem Reaktoraustrag aus Reaktor 2 der Kolonne 11 zugeführten Leichtsieder, z.B. Vinylcyclohexen, werden, gewünschtenfalls zusammen mit dem in Kolonne 11 abgetrennten Rest-Butadien, über Auslaß 26 zur Fackel geleitet. Anstelle einer einzigen Destillationskolonne 11 können auch mehrere Destillationskolonnen hintereinander geschaltet sein. Dadurch können höhersiedende, im Austrag von Reaktor 2 enthaltene Reaktionsprodukte, z.B. die Alkoxyoctadiene oder Alkoxydodecatriene, vom Addukt II abgetrennt und aus dem Verfahren ausgeschleust werden. Da diese Verbindungen sich bei der Hydrierung (d) nicht störend auswirken, kann auch eine Abtrennung unterbleiben und die Alkoxyoctadiene oder Alkoxydodecatriene mit dem Addukt II über die Leitung 13 dem Hydrierreaktor 14 zugeführt werden.

Der vom leichter flüchtigen Addukt III und von leichtsiedenden und gegebenenfalls von höhersiedenden Nebenprodukten befreite flüssige Austrag von Kolonne 11 wird gegebenenfalls mit nicht umgesetzten Alkohol ROH I über Leitung 13 dem Hydrierreaktor 14 zugeführt, wo das Addukt II in Gegenwart eines homogenen oder heterogenen Übergangsmetallkatalysators zum n-Butylalkylether IV hydriert wird. Über Leitung 15 oder 16 wird der benötigte Wasserstoff zugeführt.

Der Reaktionsaustrag aus Reaktor 14, der im wesentlichen n-Butylalkylether, höhersiedende Butadien-Derivate, beispielsweise Octylalkylether oder Dodecylalkylether sowie, falls im Reaktor 14 ein Homogenkatalysator verwendet wurde, Katalysatorlösung enthält, wird über Leitung 17 der Destillationskolonne 18 zugeführt. Nicht umgesetzter Wasserstoff wird zum größten Teil über Leitung 19 abgezogen und entweder über die Leitungen 15 oder 16 wieder in die Umsetzung zurückgeführt oder abgefackelt. Gewünschtenfalls kann der Wasserstoff auch über einen zwischen dem Reaktor 14 und der Destillationskolonne 18 angebrachten Gas-Flüssigkeit-Abscheider abgetrennt und wie vorher dargestellt, weiter verwendet werden.

In der Destillationskolonne 18 wird der Hydrieraustrag aus Reaktor 14 destillativ in seine Bestandteile aufgetrennt. Leichtsiedende Nebenprodukte werden über Kopf über Leitung 20 abgezogen und zwecks weiterer Reinigung in einer zusätzlichen Destillationsstufe aufgearbeitet, die hier nicht eingezeichnet ist. Die n-Butylalkylether werden über Leitung 21 aus der Kolonne abgeführt, höhersiedende Produkte, beispielsweise Octylalkylether und Dodecylalkylether werden über mehrere Auslässe (22) im unteren Teil der Kolonne 18 entnommen. Wurde im Reaktor 14 ein Homogenkatalysator verwendet, so wird die Katalysatorlösung aus dem Sumpf der Kolonne 18 über Leitung 23 entnommen und gegebenenfalls nach Ausschleusung eines Teilstroms an verbrauchtem Katalysator über Leitung 24 und Ergänzung mit frischer Katalysatorlösung über Leitung 25, wieder in den Reaktor 14 zurückgeführt.

Aus US 2 922 822 (Beispiel 1) und aus WO 95/19334 ist zwar schon bekannt, daß man durch Trennung der Addukte von Alkoholen an Butadien und Rückführung eine Isomerisierung durchführen kann, um so nur eines der beiden Isomere herzustellen. In beiden Schriften ist jedoch weder beschrieben noch nahegelegt, daß es durch eine Kombination von Additionsreaktion, Trennung, Isomerisierung und Hydrierung gelingt auf eine neue und technisch sehr vorteilhafte Weise Alkylbutylether herzustellen.

### Beispiele

### Beispiel 1 (Addition von Alkohol an Butadien und Isomerisierung) (Teilreaktion a)

Die Herstellung von Butylbutenylethergemisch (3-Butoxybut-1-en und 1-Butoxybut-2-en) erfolgte wie in den Beispielen 1 bis 4 sowie 8 bis 11 von WO 95/19334 beschrieben.

Die Isomerisierung von Addukt III zu Addukt II in einer getrennten Isomerisierungsstufe bzw. durch Rückführung des Addukts der Formel III in die Stufe (a) erfolgte wie in Beispiel 5 von WO 95/19334 beschrieben.

### Beispiel 2 (Hydrierung des Addukts II mittels homogener Katalyse)

a) Ein Autoklav wurde mit 0,25 g des Katalysators
   HRuCl(CO)(PPh₃)₃ und 26,95 g (210 mmol) 1-Butoxybut-2-en befüllt und 6 Stunden unter 18 bar Wasserstoffatmosphäre bei 150°C gerührt. Anschließend wurde das Reaktionsgemisch mittels kalibrierter Gaschromatographie analysiert. Bei einem Umsatz von 92 % wurde Di-n-butylether mit einer Selektivität von 90 %, 1-Butoxybut-1-en mit einer Selektivität von 3 % und 1,1-Dibutoxybutan mit einer Selektivität von 4 % erhalten.
b) Ein Autoklav wurde mit 0,24 g des Katalysators HRh(CO) (PPh₃)₃ und 26,95 g (210 mmol) 1-Butoxybut-2-en befüllt und 6 Stunden unter 18 bar Wasserstoffatmosphäre bei 150°C gerührt. Anschließend wurde das Reaktionsgemisch mittels kalibrierter Gaschromatographie analysiert. Bei einem Umsatz von 82 % wurde Di-n-butylether mit einer Selektivität von 76 %, 1-Butoxybut-1-en mit einer Selektivität von 13 % und 1,1-Dibutoxybutan mit einer Selektivität von 8 % erhalten.

### Beispiel 3 (Hydrierung des Addukts II mittels heterogener Katalyse in flüssiger Phase)

Ein Glasautoklav wurde mit 0,1 g des Katalysators Palladium auf Aktivkohle (10%) und 7,0 g (54 mmol) 1-Butoxybut-2-en befüllt. Nach 3 Stunden Reaktionszeit bei 100°C unter 12 bar Wasserstoffdruck wurde das Reaktionsgemisch mittels kalibrierter Gaschromatographie analysiert. Bei einem Umsatz von 99 % wurde Di-n-butylether mit einer Selektivität von 82 % erhalten.

### Beispiel 4 (Hydrierung des Addukts II mittels heterogener Katalyse in der Gashase)

In einen Reaktor wurden 100 ml eines Nickel/Kupfer-Aluminiumoxid-Katalysators, mit einem Nickelgehalt, berechnet als NiO, von 50 Gew.-% und einem Kupfergehalt, berechnet als CuO, von 17 Gew.-% eingefüllt. Die Temperatur des Katalysatorbettes wurde innerhalb von 2 Stunden in Gegenwart von Formiergas (2% Wasserstoff) bei Atmosphärendruck auf eine Temperatur von 200°C erhöht. Anschließend wurde der Wasserstoffgehalt nach jeweils 2 Stunden auf 25%, 50% und 100% gesteigert.

Danach wurde der Reaktor auf 170°C abgekühlt und 24 g/h 1-Butoxy-2-en bei Atmosphärendruck durch den Reaktor geleitet. Gleichzeitig wurde dem Reaktor ein Wasserstoffstrom von 9,5 l/h zugeführt. Nach Abkühlung wurde der einphasige, flüssige Reaktoraustrag mittels kalibrierter Gaschromatographie analysiert. Bei einem Umsatz von 99% wurde Di-n-butylether mit einer Selektivität von 99% gebildet.

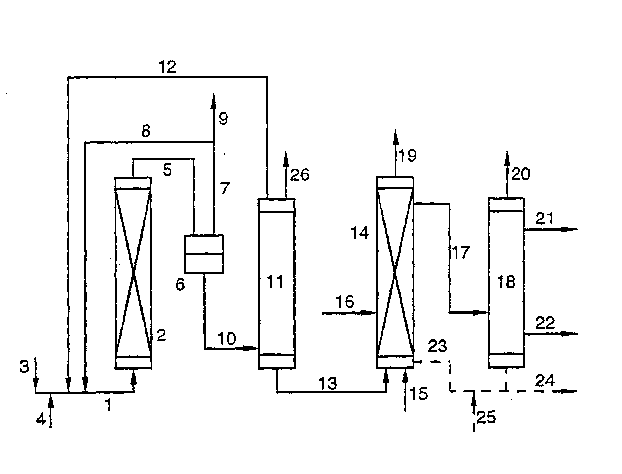

## Patentansprüche

1. Verfahren zur Herstellung n-Butylalkylethern, dadurch gekennzeichnet, daß man
a) 1,3-Butadien oder Butadien-haltige Kohlenwasserstoffgemische mit einem Alkohol der Formel I
ROH I,
in der der Rest R eine unsubstituierte oder mit 1 bis 2 C₁- bis C₁₀-Alkoxy- oder Hydroxygruppen substituierte C₂- bis C₂₀-Alkyl-, Alkenyl-, Cycloalkyl- oder Cycloalkenyl-, eine C₆- bis C₁₀-Aryl- oder eine C₇- bis C₁₁-Aralkylgruppe oder die Methylgruppe ist, bei erhöhter Temperatur und bei erhöhtem Druck in Gegenwart einer Brönsted-Säure oder in Gegenwart eines Komplexes eines Elementes aus der Gruppe Ib, VIIb oder VIIIb des Periodensystems der Elemente mit Phosphor- oder Stickstoff-haltigen Liganden zu einem Gemisch der Addukte der Formeln II 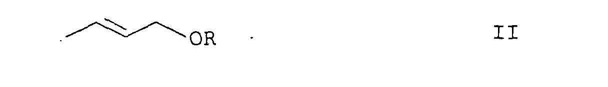 und III 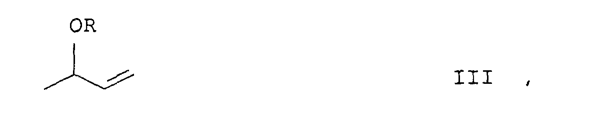 in der R die obengenannten Bedeutungen hat, umsetzt,
b) die Addukte der Formel II und III trennt,
c) das Addukt III zum Addukt II in Gegenwart eines Katalysators wie für (a) angegeben, isomerisiert, und
d) das Addukt II in Gegenwart eines homogenen oder heterogenen Übergangsmetallelement-Katalysators in flüssiger Phase oder in Gegenwart eines heterogenen Übergangsmetallelement-haltigen Katalysators in der Gasphase zum n-Butylalkylether der Formel IV 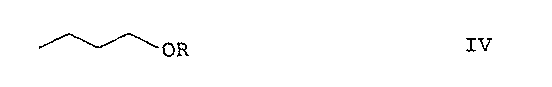 hydriert.

2. Verfahren nach Anspruch 1, dadurch gekennzeichnet, daß man die Umsetzung von 1,3-Butadien oder eines Butadien-haltigen Kohlenwasserstoffgemischs mit einem Alkohol ROH I in Gegenwart eines sauren Ionenaustauschers vornimmt.

3. Verfahren nach Anspruch 1, dadurch gekennzeichnet, daß man die Umsetzung von 1,3-Butadien oder eines Butadien-haltigen Kohlenwasserstoffgemischs mit einem Alkohol ROH I in Gegenwart eines Katalysators aus einem Alkyl-, Aryl- oder Arylalkyl-Phosphin-Komplex eines Übergangsmetalls aus der Gruppe VIIb oder VIIIb des Periodensystems der Elemente durchführt.

4. Verfahren nach Anspruch 1, dadurch gekennzeichnet, daß man die Umsetzung von 1,3-Butadien oder eines Butadien-haltigen Kohlenwasserstoffgemischs mit einem Alkohol ROH I in Gegenwart eines Katalysators aus einem Alkyl-, Aryl- oder Arylalkyl-Phosphin-Komplex des Palladiums durchführt.

5. Verfahren nach Anspruch 1, dadurch gekennzeichnet, daß man die Isomerisierung des Addukts III zum Addukt II in Gegenwart eines Katalysators durchführt, wie er zur Katalyse der Addition des Alkohols ROH I gemäß Teilreaktion (a) verwendet wird, oder daß man das Addukt III unmittelbar in die Teilreaktion (a) zurückführt und dort zum Addukt II isomerisiert.

6. Verfahren nach Anspruch 1, dadurch gekennzeichnet, daß man die Hydrierung (d) in der flüssigen Phase oder der Gasphase über einem heterogenen Hydrierkatalysator vornimmt.

7. Verfahren nach Anspruch 1, dadurch gekennzeichnet, daß man die Hydrierung (d) in der Gasphase in Gegenwart mindestens eines heterogenen Katalysators, der ein oder mehrere Elemente aus den Gruppen Ib, VIb, VIIb und/oder VIIIb des Periodensystems der Elemente enthält, durchführt.

8. Verfahren nach Anspruch 1, dadurch gekennzeichnet, daß man die Hydrierung (d) in Gegenwart mindestens eines heterogenen Katalysators, der Palladium, Nickel oder Kupfer oder Gemische dieser Elemente auf einem Trägermaterial enthält, durchführt.

9. Verfahren nach Anspruch 1, dadurch gekennzeichnet, daß man als Alkohol ROH I n-Butanol verwendet und ihn in Stufe (a) in einem mindestens einmolaren Überschuß einsetzt.

10. Verfahren gemäß Anspruch 1, dadurch gekennzeichnet, daß man die Umsetzung zumindest in den Stufen (a) bis (c) in Gegenwart von bis zu 20 Gew.-% Wasser, bezogen auf das Reaktionsgemisch durchführt.

## Revendications

1. Procédé de préparation d'éthers alkyliques de n-butyle, caractérisé en ce que l'on fait réagir
a) le 1,3-butadiène ou des mélanges d'hydrocarbures contenant un butadiène, avec un alcool de formule I
ROH I,
dans laquelle le résidu R est un groupe alkyle en C₂ à C₂₀, alcényle, cycloalkyle ou cycloalcényle, non substitué ou bien substitué par 1 à 2 groupes alcoxy en C₁ à C₁₀ ou par des groupes hydroxy, un groupe aryle en C₆ à C₁₀, ou un groupe aralkyle en C₇ à C₁₁, ou le groupe méthyle, à une température élevée et à une pression élevée en présence d'un acide de Brønsted ou en présence d'un complexe d'un élément choisi dans le Groupe Ib, VIIb ou VIIIb du Tableau Périodique des Eléments avec des ligands contenant du phosphore ou de l'azote; en obtenant un mélange des produits d'addition de formules II 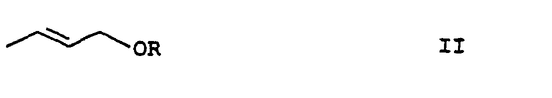 et III 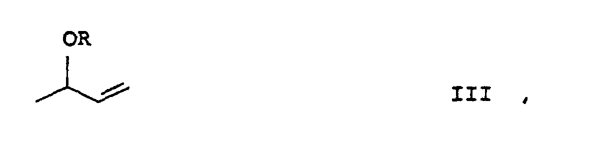 dans lesquelles R prend les significations précitées,
b) l'on sépare les produits d'addition de formule II et III,
c) l'on fait subir une isomérisation au produit d'addition III en obtenant le produit d'addition II en présence d'un catalyseur tel que celui indiqué pour (a), et
d) l'on fait subir une hydrogénation au produit d'addition II en présence d'un catalyseur homogène ou hétérogène à élément des métaux de transition, en phase liquide ou en présence d'un catalyseur hétérogène contenant un élément des métaux de transition, en phase gazeuse en obtenant l'éther alkylique de n-butyle de formule IV

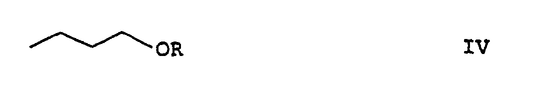

2. Procédé selon la revendication 1, caractérisé en ce que l'on procède à la réaction du 1,3-butadiène ou d'un mélange d'hydrocarbures contenant un butadiène avec un alcool ROH I en présence d'un échangeur d'ions acide.

3. Procédé selon la revendication 1, caractérisé en ce que l'on réalise la réaction du 1,3-butadiène ou d'un mélange d'hydrocarbures contenant un butadiène avec un alcool ROH I en présence d'un catalyseur constitué d'un complexe alkyl-, aryl-, ou arylalkyl-phosphine d'un métal de transition du Groupe VIIb ou VIIIb du Tableau Périodique des Eléments.

4. Procédé selon la revendication 1, caractérisé en ce que l'on réalise la réaction du 1,3-butadiène ou d'un mélange d'hydrocarbures contenant du butadiène avec un alcool ROH I en présence d'un catalyseur constitué d'un complexe alkyl-, aryl- ou arylalkyl-phosphine du palladium.

5. Procédé selon la revendication 1, caractérisé en ce que l'on réalise l'isomérisation du produit d'addition III en produit d'addition II en présence d'un catalyseur, de la façon dont il est utilisé pour la catalyse de l'addition de l'alcool ROH I selon la réaction partielle (a), ou en ce que l'on recycle directement le produit d'addition III dans la réaction partielle (a) et que l'on lui y fait subir une isomérisation en produit d'addition II.

6. Procédé selon la revendication 1, caractérisé en ce que l'on procède à l'hydrogénation (d) en phase liquide ou en phase gazeuse sur un catalyseur hétérogène d'hydrogénation.

7. Procédé selon la revendication 1, caractérisé en ce que l'on réalise l'hydrogénation (d) en phase gazeuse en présence d'au moins un catalyseur hétérogène, qui contient un ou plusieurs éléments des Groupes Ib, VIb, VIIb et/ou VIIIb du Tableau Périodique des Eléments.

8. Procédé selon la revendication 1, caractérisé en ce que l'on réalise l'hydrogénation (d) en présence d'au moins un catalyseur hétérogène qui contient le palladium, le nickel ou le cuivre ou des mélanges de ces éléments sur un matériau support.

9. Procédé selon la revendication 1, caractérisé en ce que l'on utilise en tant qu'alcool ROH I, le n-butanol et on le met en oeuvre dans l'étape (a) dans un excès au moins monomolaire.

10. Procédé selon la revendication 1, caractérisé en ce que l'on réalise la réaction au moins dans les étapes (a) à (c) en présence de jusqu'à 20% en poids d'eau, par rapport au mélange réactionnel.

## Claims

1. A process for preparing n-butyl alkyl ethers which comprises
a) reacting 1,3-butadiene or a butadiene-containing hydrocarbon mixture with an alcohol of the formula I
ROH I,
where the radical R is a C₂-C₂₀-alkyl, alkenyl, cycloalkyl or cycloalkenyl group, each of which may be unsubstituted or substituted by 1 or 2 C₁-C₁₀-alkoxy or hydroxy groups, a C₆-C₁₀-aryl group or a C₇-C₁₁-aralkyl group or the methyl group, at elevated temperature and elevated pressure in the presence of a Brönsted acid or in the presence of a complex of an element from group Ib, VIIb or VIIIb of the Periodic Table of the Elements with phosphorus- or nitrogen-containing ligands to give a mixture of adducts of the formulae II 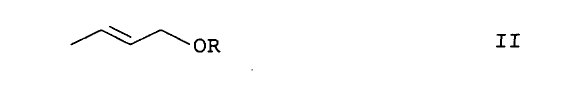 and III 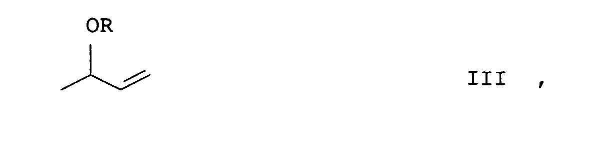 where R is as defined above,
b) separating the adducts of the formula II and III,
c) isomerizing the adduct III in the presence of a catalyst as indicated for (a) to give the adduct II and
d) hydrogenating the adduct II in the liquid phase in the presence of a homogeneous or heterogeneous transition metal catalyst or in the gas phase in the presence of a heterogeneous transition metal-containing catalyst to give an n-butyl alkyl ether of the formula IV

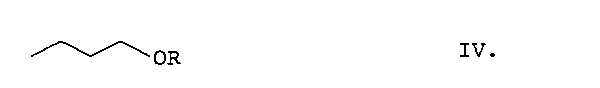

2. A process as claimed in claim 1, wherein the reaction of 1,3-butadiene or a butadiene-containing hydrocarbon mixture with an alcohol ROH I is carried out in the presence of an acid ion exchanger.

3. A process as claimed in claim 1, wherein the reaction of 1,3-butadiene or a butadiene-containing hydrocarbon mixture with an alcohol ROH I is carried out in the presence of a catalyst comprising an alkylphosphine, arylphosphine or arylalkylphosphine complex of a transition metal from group VIIb or VIIIb of the Periodic Table of the Elements.

4. A process as claimed in claim 1, wherein the reaction of 1,3-butadiene or a butadiene-containing hydrocarbon mixture with an alcohol ROH I is carried out in the presence of a catalyst comprising an alkylphosphine, arylphosphine or arylalkylphosphine complex of palladium.

5. A process as claimed in claim 1, wherein the isomerization of the adduct III to give the adduct II is carried out in the presence of a catalyst as is used for catalyzing the addition of the alcohol ROH I according to the subreaction (a), or the adduct III is returned directly to the subreaction (a) and is isomerized there to give the adduct II.

6. A process as claimed in claim 1, wherein the hydrogenation (d) is carried out in the liquid phase or the gas phase over a heterogeneous hydrogenation catalyst.

7. A process as claimed in claim 1, wherein the hydrogenation (d) is carried out in the gas phase in the presence of at least one heterogeneous catalyst comprising one or more elements from the groups Ib, VIb, VIIb and/or VIIIb of the Periodic Table of the Elements.

8. A process as claimed in claim 1, wherein the hydrogenation (d) is carried out in the presence of at least one heterogeneous catalyst comprising palladium, nickel or copper or a mixture of these elements on a support material.

9. A process as claimed in claim 1, wherein the alcohol ROH I used is n-butanol and is used in stage (a) in an at least one molar excess.

10. A process as claimed in claim 1, wherein the reaction, at least in stages (a) to (c), is carried out in the presence of up to 20 % by weight of water, based on the reaction mixture.
